# EUROPEAN PATENT APPLICATION

(11) **EP 0 987 330 A1**
(43) Date of publication of application: **22.03.2000**
(21) Application number: 98116492.4
(22) Date of filing: 01.09.1998
(51) Int. Cl.: C12N 15/82, C12N 9/14

(54) **Modification of plant development and plant differentiation by use of tissue specific Deac gene expression system**

(71) Applicant: Hoechst Schering AgrEvo GmbH, 13509 Berlin (DE)
(72) Inventor: Bartsch, Klaus Dr., 61462 Königstein (DE)

(57) **Abstract**

Instant invention relates to a method for the modification or delay of meristem differentiation and/or development by tissue- and/or developmental-specific expression of a deacetylase gene (deac) encoding a protein exhibiting N-acetyl-PPT (e.g., N-acetyl-phosphinothricine) specific deacetylase activity in combination with the application of a composition containing one or more non-toxic compound(s) (e.g., N-acetyl-PPT), allowing - after deacetylation within the plant cell - inducible and selective modification of meristem cells within a living plant in order to modify differentiation and/or development of the plant.

## Description

Instant invention relates to a method for the modification or delay of plant development or plant tissue differentiation by tissue- and/or developmental-specific expression of a deacetylase gene (deac) encoding a protein exhibiting N-acetyl-PPT (e.g., N-acetyl-phosphinothricine) specific deacetylase activity (DEAC) in combination with the application of a composition containing one or more non-toxic compound(s) (e.g., N-acetyl-PPT), allowing - after deacetylation within the plant cell - inducible and selective modification of cell differentiation within a living plant.

One of the main problems of plant protection in agriculture is the avoidance of yield loss. A specific problem in this regard is the appropriate time point of hervesting for the farmer dependent on environmental facters, e.g., wheather conditions such as drought, rain, etc..

In plants, the produced photoassimilates from green leaves (source organs) are used either for vegetative and generative growth or are stored in tubers, seeds and fruits (sink organs).

The yield of plants with root or shoot tubers (e.g., sugar beet, potato) is reduced after inflorescence induction (bolting), since assimilates from storage organs and leaves are utilized for flower development and seed production. On the other hand, bolting is a problem in many crops and legumes, which affects yield quantity and quality. Thus, inhibition or delay of flowering and shoot elongations result in a prolonged productivity period and increased storage yield.

However, up to now, an external suppression of flowering in crop plants is not possible.

Therefore, it is an object of the present invention to provide a solution to the above-mentioned problem of external control of plant differentiation which allows the farmer to choose flexibly the appropriate time point for harvesting without having the disadvantage with respect to yield loss caused by undesired flowering, bolting and the like.

This object has been achieved by use of the deac system according to the invention and by the provision of the embodiments described herein.

The use of the deac system for the selective ablation of plant tissue and cells is known from EP-A-0 531 716. Depending on the type of promoter used, specific cell ablation can be achieved for any plant tissue of interest, e.g., for the induction of transgenic male or female sterility by anther or pistil specific expression of a deacetylase gene. By fusing a deac gene with a tapetum-specific promoter male sterility (MS) can be induced by external application of N-acetyl-phosphinothricine. The N-acetyl-phosphinothricine-specific deacetylase protein (DEAC), the expression product of the deac gene, converts the non-toxic N-acetyl-derivative of phosphinothricine into the herbicidal active compound (WO 98/27201).

Genes coding for suitable deacetylase proteins (DEAC) with appropriate substrate specificities have been described previously: *argE* from *Escherichia coli*, encoding N-acetyl-ornithine deacetylase, *dea* from *Streptomyces viridochromogenes* (EP-A-0 531 716), *deac1* from *Stenotrophomonas sp.* (DSM 9734) and *deac2* from *Comamonas acidovorans* (DSM 11070), both encoding hippurate hydrolases (WO 98/27201).

Now, it has been surprisingly found that the use of the deac system, i.e. the expression of a deac gene under control of a meristem specific promoter within a specific plant tissue (e.g., vegetative or generative meristem) in combination with the external application of N-acetyl PPT is suitable for the modification or delay of plant development and differentiation by selective manipulation of the specific plant cells or tissues in order to optimize agricultural productivity in dependance from environmental growth conditions.

The reaction induced by the deac system according to the invention does not result in a general death of the apical meristem, but acts by inhibiting meristem differentiation, thereby delaying flowering or bolting.

Surprisingly, the effects caused by the deac system are locally limited to the target tissue and can be fine tuned, depending on the expression level of the DEAC and the concentration of N-acetyl PPT in a specific tissue. Thus, choosing the proper promoter, alteration or modification in plant phenotype is achievable.

Because of the known problem that many tissue- or development-specific plant promoters are *"*leaky*",* i.e., that they are also expressed to a certain extent in other tissues or developmental stages or are also inducible by other environmental stress factors, they are not suitable for most purposes.

Using the deac system according to the invention the obstacle of those *"*leaky*"* promoters is overcome, since the deac system is only switched on after application of N-acetyl PPT, a non-toxical chemical.

Induction of flowering in plants takes place as response to internal and external cues and is initiated by transformation of the vegetative into a generative meristem giving rise to inflorescence and floral development. A number of flowering mutants have been characterized especially from *Arabidopsis thaliana* and *Antirrhinum majus* and several genes with specific expression in inflorescence and floral meristems have been cloned (e.g. AtK-1: Jonak C. et al., 1995, Plant Mol. Biol. 27:217-221, Dockx, J. et al., 1995, Plant Mol. Biol. 28:723-737, AGL8: Mandel, M. A. and Yanofsky, M. F., 1995, The Plant Cell 7:1763-1771, AP1: Mandel et al., 1992, Nature 360:273-277).

In some cases also the corresponding promoters have been characterized and tissue-specific expression was demonstrated by GUS-reporter gene fusions.

Another reason for reduced yield in crop plants grown in monoculture is the so called "shade avoidance response" (Quail, P. H., 1996, Nature Biotechnology 14:945). Due to perceived competition for light, plants respond with increased stem extension growth at the expense of harvestable compounds such as leaves, storage tissue or reproductive tissue. In this case transgenic crops could be generated expressing the deac gene under control of a promoter specific for the vegetative meristem (e.g. KNAT1, KNAT2; Lincoln, C. et al., 1994, The Plant Cell 6:1859-1876). Treatment of those plants with N-acetyl-PPT will damage the meristemic cells leading to reduced shoot elongation in favour of further leaf development, probably with an accompanying increase in harvest index.

The problem to suppress externally plant cell differentiation, e.g. flowering or bolting in plants is addressed by instant invention expressing a deac gene under control of a meristem specific, e.g., vegetative or generative meristem specific promoter in a transgenic plant. Treatment of those plants with N-acetyl-PPT just before or during deac expression in the meristem results in modification or delay of differentiation of the induced cells. As a consequence bolting and flower development will be inhibited, resp., delayed as long as the compound is available in the meristem.

In phosphinothricin-tolerant plants, which express the phosphinothricin acetyltransferase (*pat*) gene, bolting or flowering control is also achievable by herbicide treatment with PPT. In this case N-acetyl-PPT is generated by the plant itself, and is selectively reconverted to the active herbicide only in the tissue expressing the deac gene.

Inhibition or delay of flowering is controlled by timing, number and concentration of applied N-acetyl-PPT, resp., PPT.

Without treatment of N-acetyl-PPT and/or PPT or when the concentration of N-acetyl-PPT and/or PPT falls below a certain level, plants will still flower and set seed and thus cross breeding and harvest of seeds is possible.

Therefore, in a first aspect the present invention relates to a nucleic acid molecule comprising the coding region of, resp., a nucleotide encoding a N-acetyl-PPT specific deacetylase under control of a meristem specific regulatory element, such as a promoter or a promoter in combination with an enhancer.

Additionally, an object of the invention is the use of a deac gene or a nucleic acid molecule of the invention for the preparation of a transgenic plant cell or plant, accessible to externally delaying meristem differentiation in order to suppress, e.g., flowering or bolting.

The nucleic acid molecule according to the invention is preferably a DNA or RNA molecule, for instance genomic or cDNA. The nucleotides coding for a N-acetyl-PPT specific deacetylase and/or a meristem specific promoter, which are part of the nucleic acid molecule of the invention, may be isolated from natural sources or synthesized by already known methods (Sambrook et al., Molecular Cloning, A Laboratory Manual, 2. Aufl. (1989) Cold Spring Harbor Laboratory Press, Cold Spring Harbor, NY).

The nucleic acid molecule according to the invention may also comprise a fragment, derivative or allelic variant of said nucleotide coding for a N-acetyl-PPT specific deacetylase provided that the biological activity of a N-acetyl-PPT specific deacetylase is restored.

Further, the nucleic acid molecule according to the invention may also comprise fragments, derivatives and allelic variants of a meristem specific promoter provided that the biological activity of a meristem specific promoter is restored.

The fragments, derivatives and allelic variants of said nucleotides coding for a N-acetyl-PPT specific deacetylase or a meristem specific promoter might have been caused by one or more mutations such as deletions, substitutions, insertions and/or recombinations, which may have naturally occured, or which may have been induced by genetic engineering or molecular biological techniques (e.g., Sambrook et al., loc.cit.).

In another embodiment the nucleic acid molecule according to invention is linked to one or more regulatory elements, which ensure transcription and synthesis of a translatable RNA in procaryotic or eucaryotic cells (e.g., enhancer, tissue-specific elements, 3'-poly A-tail, e.g., Gielen et al., EMBO J. 1989, 8:23-29).

When expressing a nucleic acid molecule according to the invention in a plant cell, in general, the expression product, i.e. the deacetylase protein, can be localized in any compartment (e.g., cytosol, vacuole, apoplast, cell wall, plastid, mitochondrium etc.) of the transformed plant cell. In order to target the expression product into a specific compartment, it is required to add to the coding sequence of said nucleic acid molecule a nucleotide coding for a signal peptide, resp., targeting sequence. Such a nucleotide coding for a signal peptide, resp., targeting sequence is well known in the art (e.g., Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Furthermore, the invention relates to vectors, preferably plasmids, cosmids, viruses, bacteriophages, and other vectors common in genetic engeneering, which comprise a nucleic acid molecule according to invention.

In a further embodiment the invention relates to a host cell, preferably procaryotic or eucaryotic cell, comprising a nucleic acid molecule or a vector according to the invention and the progeny of said host cell.

The host cell of the invention is characterized in that it has been transformed and/or genetically modified by a nucleic acid molecule according to the invention or by a vector according to the invention. The progeny of said host cell is further characterized by the presence of the nucleic acid molecule according to the invention. Preferably, the host cell is a bacterial cell. The host cell of the invention is further characterized in that the introduced nucleic acid molecule is of heterologous origin, i.e. it does not occur naturally in these cells.

Moreover, the present invention relates to a process for the production of a transgenic plant cell comprising the step of introducing a nucleic acid molecule or a vector according to the invention into the genome of a plant cell by known transformation technologies, e.g., by stable integration into the genome of said plant cell, said transgenic plant cell expressing a N-acetyl-PPT specific deacetylase under control of a meristem specific regulatory element.

The transformed plant cell obtainable by the process of the invention is a further embodiment of the invention. The plant cell according to the invention expresses a N-acetyl-PPT specific deacetylase under control of a meristem specific promoter and is further characterized in that the introduced nucleic acid molecule is of heterologous origin, i.e. it does not occur naturally in these cells.

In another embodiment of the invention the transformed plant cell of the invention additionally expresses a nucleic acid molecule conferring PPT-resistance as known, e.g., by EP-A-0 242 236, EP-A-0 242 246, EP-A-0 257 542 or EP-A-0 531 716, obtainable, e.g., by introducing a known PPT-resistant or tolerant plant cell into the process according to the invention.

By means of methods known to the skilled person in the art the transgenic plant cell of the invention can be regenerated to a complete plant.

Therefore, a further object of the invention is a process for the production of a transgenic plant comprising the step of regenerating a complete plant from a plant cell according to invention, said plant exhibiting modified meristem differentiation and/or development after treatment with N-acteyl-PPT. Such a plant is obtainable by introducing a plant cell of the invention into a well known process for plant regeneration. If a plant cell comprising a gene conferring PPT-resistance (e.g., a pat or bar gene) is transformed with a nucleic acid molecule or a vector according to the invention, such a plant exhibits modified meristem differentiation and/or development after treatment with either N-acetyl-PPT and/or PPT.

Accordingly, if a plant according to the invention exhibits additionally PPT-tolerance, the treatment of said plant with PPT results simultaneously in a herbicidal effect and modification or delay of meristem differentiation and/or development.

In order to prepare the integration of foreign nucleic acid molecules into higher plants a high number of cloning vectors are available, containing a replication signal for E.coli and a marker gene for the selection of transformed bacterial cells. Examples of such vectors are pBR322, pUC series, M13mp series, pACYC184, pBinAR etc. The desired sequence may be integrated into the vector at a suitable restriction site. The obtained plasmid is used for the transformation of E.coli cells. Transformed E.coli cells are cultivated in a suitable medium and subesequently harvested and lysed. The plasmid is recovered. As an analyzing method for the characterization of the obtained plasmid DNA, use is generally made of restriction analysis, gel electrophoresis and other biochemical or molecularbiological methods. After each manipulation the plasmid DNA may be cleaved and the obtained DNA fragments may be linked to other DNA sequences. Each plasmid DNA may be cloned into the same or other plasmids. In order to integrate DNA into plant host cells, a wide range of techniques are available. These techniques comprise the transformation of plant cells with T-DNA by using Agrobacterium tumefaciens or Agrobacterium rhizogenes as transformation medium, by polyethylene glycol treatment, the fusion of protoplasts, the injection or electroporation of DNA, the integration of DNA by means of the biolistic method as well as further possibilities.

In the case of injection and electroporation of DNA into plant cells, there are no special demands made on the plasmids used. Simple plasmids such as pUC derivatives may be used. However, in case that whole plants are to be regenerated from cells transformed in such a way, a selectable marker gene should be present.

Depending on the method of integrating desired genes into the plant cell, further DNA sequences may be necessary. If the Ti- or Ri-plasmid is used e.g. for the transformation of the plant cell, usually at least the right border, more frequently, however, the right and left border of the Ti- and Ri-plasmid T-DNA should be connected to the foreign gene to be integrated as a flanking region.

If Agrobacteria are used for the transformation, the DNA which is to be integrated should be cloned into special plasmids, namely either into an intermediate vector or into a binary vector. Due to sequences homologous to the sequences within the T-DNA, the intermediate vectors may be integrated into the Ti- or Ri-plasmid of the Agrobacterium due to homologous recombination. This also contains the vir-region necessary for the transfer of the T-DNA. Intermediate vectors cannot replicate in Agrobacteria. By means of a helper plasmid the intermediate vector may be transferred to Agrobacterium tumefaciens (conjugation). Binary vectors may replicate in E.coli as well as in Agrobacteria. They contain a selectable marker gene as well as a linker or polylinker which is framed by the right and the left T-DNA border region. They may be transformed directly into Agrobacteria (Holsters et al. Mol. Gen. Genet. 163 (1978), 181-187). The Agrobacterium acting as host cell should contain a plasmid carrying a vir-region. The vir-region is usually necessary for the transfer of the T-DNA into the plant cell. Additional T-DNA may be present. The Agrobacterium transformed in such a way is used for the transformation of plant cells.

The use of T-DNA for the transformation of plant cells was investigated intensively and described sufficiently in EP 120 516; Hoekema, In: The Binary Plant Vector System Offsetdrukkerij Kanters B.V., Alblasserdam (1985), Chapter V; Fraley et al., Crit. Rev. Plant. Sci., 4, 1-46 and An et al. EMBO J. 4 (1985), 277-287.

For transferring the DNA into the plant cells, plant explants may suitably be co-cultivated with Agrobacterium tumefaciens or Agrobacterium rhizogenes. From the infected plant material (e.g. pieces of leaves, stem segments, roots, but also protoplasts or suspension-cultivated plant cells) whole plants may then be regenerated in a suitable medium which may contain antibiotics or biocides for the selection of transformed cells. The plants obtained in such a way may then be examined as to whether the integrated DNA is present or not. Other possibilities in order to integrate foreign DNA by using the biolistic method or by transforming protoplasts are known to the skilled person (cf. e.g. Willmitzer, L., 1993 Transgenic plants. In: Biotechnology, A Multi-Volume Comprehensive Treatise (H.J. Rehm, G. Reed, A. Pühler, P. Stadler, editors), Vol. 2, 627-659, VCH Weinheim-New York-Basel-Cambridge).

Alternative systems for the transformation of monocotyledonous plants are transformation by means of a biolistic approach, the electrically or chemically induced DNA integration in protoplasts, the electroporation of partially permeabilized cells, the macro-injection of DNA into inflorescences, the micro-injection of DNA into microspores and pro-embryos, the DNA integration by sprouting pollen and the DNA integration in embryos by swelling (review given in: Potrykus, Physiol. Plant (1990), 269-273).

Whereas the transformation of dicotyledonous plants by Ti-plasmid-vector systems by means of Agrobacterium tumefaciens is a well-established method, more recent studies indicate that the transformation with vectors based on Agrobacterium can also be used in the case of monocotyledonous plants (Chan et al., Plant Mol. Biol. 22 (1993), 491-506; Hiei et al., Plant J. 6 (1994), 271-282; Bytebier et al., Proc. Natl. Acad. Sci. USA 84 (1987), 5345-5349; Raineri et al., Bio/Technology 8 (1990), 33-38; Gould et al., Plant Physiol. 95 (1991), 426-434; Mooney et al., Plant, Cell Tiss. & Org. Cult. 25 (1991), 209-218; Li et al., Plant Mol. Biol. 20 (1992), 1037-1048, Jähne et al., Euphytica 85 (1995), 35-44, Maheshwari et al., Critical Reviews in Plant Science 14 (2) (1995), 149-178, Hess et al. (Plant Sci. 72 (1990), 233, Vasil et al., (Bio/Technology 10 (1992), 667-674, Weeks et al. (Plant Physiol. 102 (1993), 1077-1084, Becker et al. (Plant J. 5 (2) (1994), 299-307.

Once the introduced DNA has been integrated in the genome of the plant cell, it usually continues to be stable there and also remains within the descendants of the originally transformed cell. It usually contains a selectable marker which confers resistance against a biocide such as phosphinotricine or against an antibiotic such as kanamycin, G 418, bleomycin or hygromycin etc. to the transformed plant cells. The individually selected marker should therefore allow for a selection of transformed cells to cells lacking the integrated DNA.

The transformed cells grow in the usual way within the plants (see also McCormick et al., Plant Cell Reports 5 (1986), 81-84). The resulting plants can be cultivated in the usual way and cross-bred with plants having the same transformed genetic heritage or another genetic heritage. The resulting hybrid individuals have the corresponding phenotypic properties. The plant cells produce seeds.

Two or more generations should be grown in order to ensure whether the phenotypic feature is kept stably and whether it is transferred. Furthermore, seeds should be harvested in order to ensure that the corresponding phenotype or other properties will remain.

Another object of the invention is the plant obtainable by a process of the invention, said plant comprising a plant cell of the invention. The plant according to the invention confers modified meristem differentiation and/or development after treatment with N-acetyl PPT and/or PPT. Preferably, the plant of the invention is an angiosperm, gymnosperm, monocotyledonous or dicotyledonous plant, especially a crop plant, in particular a plant for the production of fruits, nuts, vegetables, fodder, cereals, ornamentals, herbs, etc., such as, Dilleniidae, Rosanae, Fabaceae, Anacardiaceae, Euphorbiaceae, Hamamelididae, Solananaceae, Pooideae, especially selected from the group consisting of rye, barley, oat, maize, wheat, millet, cassava, sago, rice, lens, pea, maniok, potato, tobacco, tomato, rape, canola, soya, sugar beet, sugar cane, hemp, cotton, flax, sunflower, mungbean, bean, banana, arrowroot, peanut, broccoli, cucumber, cauliflower, cabbage, lettuce, paprika, apricot, peach, plum, cherry, apple, pear, quince, tea, coffee, orange, lemon, grapefruit, grape, gooseberry, blackberry, cranberry, raspberry, strawberry, blueberry, lupin, clover, vicia faba, chickpea and cashew.

The progeny of said plant or plant cell comprises fruits, seeds, tubers, root-stocks, seedlings, cuttings, calli, cell cultures etc. which are characterized by the presence of the nucleic acid molecule according to the invention.

Another object of the invention is a process for the modification or delay of meristem differentiation and/or development in plants according to the invention, wherein N-acetyl-PPT and/or PPT is applied to said plants.

Eventually, it is an object of the invention to use the deac system for the modification or delay of meristem differentiation and/or development, and also to use N-acetyl-PPT and/or PPT for the modification or delay of meristem differentiation and/or development in plants of the invention.

Since the deac system according to the invention offers an optional solution (an option for the modification or delay of meristem differentiation and/or development depending on the application of N-acetyl-PPT, resp., PPT), it can be used to optimize agricultural productivity dependent from environmental conditions influenced by soil, light, temperature, drought, humidity, stress, etc.. In the case that no change in the development of the crops is desired, N-acetyl-PPT, resp., PPT need not to be applied.

In this application, the term *"*deac system*"* shall have the meaning of one or more gene(s) coding for a protein exhibiting deacetylase activity under control of a meristem specific promoter in combination with the application of one or more non-toxic compounds which act as substrate(s) of said deacetylase(s) or compositions comprising said non-toxic compounds, which - after deacetylation - lead to a limited toxic effect within a plant tissue, plant cell and/or developmental stage of the plant.

The term "deacetylase activity" shall have the meaning of an enzymatic acitivity with a specifity for N-acetylated PPT (N-acetyl-PPT).

The term "PPT" is not restricted to the herbicidal active compound phosphinothricine (2-amino-4-methylphosphinobutyric acid, glufosinate), but shall also comprise derivatives and related compounds such as desmethylphosphinothricine (2-amino-4-hydroxyphosphinobutyric acid), bialaphos (phosphinothricyl-alanyl-alanine), desmethylbialaphos and the like, their salts, racemic mixtures and active enantiomers.

In PPT-tolerant plants, which express, e.g., the phosphinothricin acetyltransferase (*pat* or *bar*) gene, the modification or delay of meristem differentiation and/or development is also achievable by herbicide treatment with PPT. In this case N-acetyl-PPT is generated by the plant itself, and is selectively reconverted to the active herbicide only in the tissue expressing the deac gene.

Transgenic plants are generated, expressing a deac gene under control of a meristem specific promoter. The developement of the plants is not affected under normal and under stress conditions, since the deac protein is not toxic to the cells per se.

The plants are monitored for meristem differentiation and/or development and the chemical, e.g., N-acetyl-PPT or PPT is applied just in time before meristem differentiation such as shooting, flowering, or bolting becomes manifest.

The term "meristem" shall have the meaning of any meristematic tissue, preferably, vegetative or generative meristem, in particular shooting, rooting, flowering or infloreszens meristem

In addition, the specific deac gene expression in combination with application of N-acetyl-PPT may be combined with other transgenic strategies for plant improvement, e.g., pathogen resistance or other molecularbiological approaches including qualitative and/or quantitative modification/alteration of plant products, e.g., by cross breeding or transformation technology.

### Example 1:

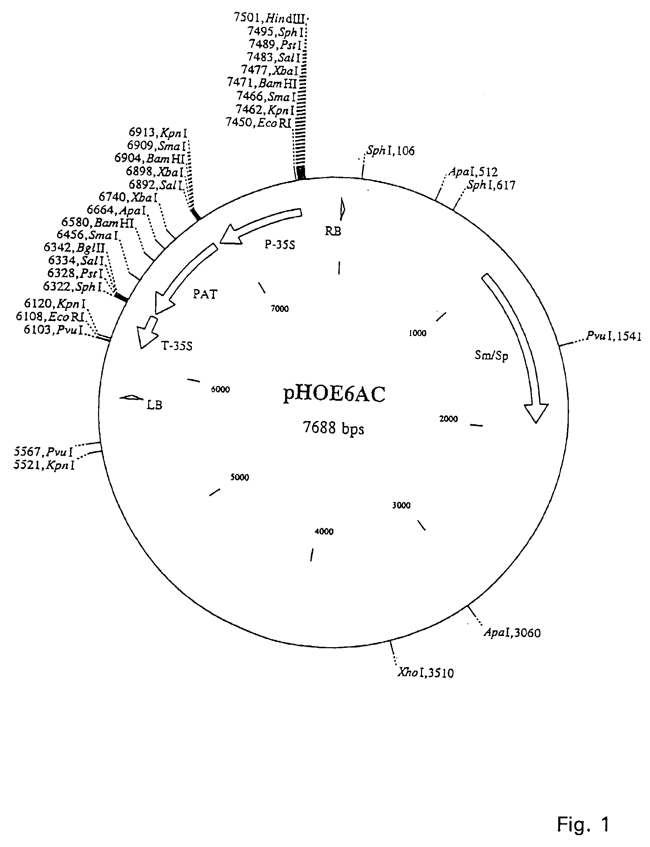

The deac 1 gene (WO 98/27201) was fused with the promoter of the AGL8 MADS box gene derived from *Arabidopsis thaliana*, which is active in the early inflorescence meristem as soon as the plant switches from vegetative to reproductive development (Mandel Yanofsky, 1995, The Plant Cell 7: 1763-1771).The promoter-gene cassette was subcloned in to the binary vectors pPCV801 (selectable marker: Pnos-*nptll*, Koncz and Schell 1986, Mol. Gen. Genet. 204: 383-396) and pHOE6AC (selectable marker: P35S-*pat*, see Fig. 1). These constructs were transformed into tobacco (*Nicotiana tabacum*) by *Agrobacterium* mediated gene transfer according to the method of Horsch et al., 1985, Science 227: 1229-1231.

Transformed plants were selected on kanamycin or phosphinothricin medium, depending on the plasmid construct, and tested for presence of the transgene by southern blot analysis.

20 plants from each transformation experiment were chosen for enzyme testing and spraying assays. From each primary transformant at least 2 plants were generated and planted into soil. DEAC activities were measured in crude extracts from apical meristems after inflorescence induction as described in DE 196 52 284. Replica plants of the transformants with the highest specific DEAC activities were selected for spraying experiments with N-acetyl-PPT.

Transgenic plants were sprayed 2x either with 5 mg/ml N-acetyl-D,L-PPT (pPGV801 -transformants) or with 2 mg/ml D,L-PPT (pHOE6AC-transformants) 1-2 weeks prior to inflorescence induction. Untreated transformants and sprayed non transgenic plants served as controls. In both groups of deac plants inflorescence development and flowering were delayed by ca. 3-4 weeks compared to the plants of the control groups.

### Example 2

The deac1 structural gene was fused with the KNAT1 promoter from Arabidopsis thaliana with specific activity for the vegetative shoot meristem (Lincoln et al., 1994, The Plant Cell, 6:1859-1876). Subcloning into the binary vectors p PCV801 and pHOE6AC as well as tobacco transformation with the final constructs were carried out as described in Example 1.

Transgenic plants were selected on kanamycin or phosphinothricine, respectively, and were checked for presence of the deac gene by southern blot analysis. From each positive primary transformant, 2 plants were regenerated and planted into soil for further analysis.

20 plants from each transformation experiment were tested for deac activity by measuring the enzymatic activity in crude extracts from vegetative shoot meristems as described (WO 98/27201).

The replica plants of deac positive transformants were chosen for spraying experiments with N-acetyl-PPT.

Plants were sprayed in groups of 5 in the 4 leaf stage 2-times per week either with 5 mg/ml N-acetyl-D,L-PPT (pPCV801-transformants) or with 2 mg/ml D,L-PPT (pHOE6AC-transformants). Untreated transformants and sprayed non transgenic plants served as controls. Shoot elongation was monitored over a total period of 4 weeks. A significant reduction in growth rate (ca. 50%) was observed in both groups of deac plants compared to the plants of the control groups.

## Claims

1. Nucleic acid molecule comprising a nucleotide encoding a N-acetyl-PPT specific deacetylase under control of a meristem specific regulatory element.

2. Nucleic acid molecule comprising a nucleotide encoding a N-acetyl-PPT specific deacetylase under control of a meristem specific promoter.

3. Nucleic acid molecule according to one or more of claims 1 to 2 which is DNA or RNA.

4. Vector comprising a nucleic acid molecule according to one or more of claims 1 to 3.

5. Host cell comprising a nucleic acid molecule according to one or more of claims 1 to 3 or a vector according to claim 4 and the progeny thereof.

6. A process for the production of a transgenic plant cell comprising the step of introducing a nucleic acid molecule according to one or more of claims 1 to 3 or a vector according to claim 4 into the genome of a plant cell, said transgenic plant cell expressing a N-acetyl-PPT specific deacetylase under control of a meristem specific regulatory element.

7. A process according to claim 6 characterized in that the plant cell is PPT-tolerant.

8. Plant cell obtainable by a process according to claim 6 or 7 characterized by expressing a N-acetyl-PPT specific deacetylase under control of a meristem specific regulatory element.

9. A process for the production of a transgenic plant comprising the step of regenerating a complete plant from a plant cell according to claim 8.

10. Plant obtainable by the process according to claim 9, characterized in that the plant comprises a plant cell according to claim 8.

11. Plant according to claim 10 which is a crop plant.

12. Progeny of a plant according to one or more of claims 10 to 11.

13. Use of a nucleic acid molecule according to one or more of claims 1 to 3, a vector according to claim 4 or a host cell according to claim 5 for the preparation of a transgenic plant cell or plant, which is accessible to the modification or delay of meristem differentiation and/or development by treatment of N-acetyl PPT and/or PPT.

14. Use of the deac gene for the preparation of a plant cell or plant, which is accessible to the modification or delay of meristem differentiation and/or development by treatment of N-acetyl PPT and/or PPT.

15. Process for the modification or delay of meristem differentiation and/or development in plants according to one or more of claims 8 to 10, characterized by application of N-acetyl-PPT and/or PPT to said plants.

16. Use of the deac system for the modification or delay of meristem differentiation and/or plant development.

17. Use of N-acetyl-PPT and/or PPT for the modification or delay of meristem differentiation and/or plant development in a plant according to one or more of claims 10 to 11.
